# EUROPEAN PATENT APPLICATION

(11) **EP 1 782 842 A1**
(43) Date of publication of application: **09.05.2007**
(21) Application number: 05077407.4
(22) Date of filing: 19.10.2005
(51) Int. Cl.: A61L 9/12

(54) **Air freshener device**

(71) Applicant: Sara Lee/DE N.V., 3532 AA Utrecht (NL)
(72) Inventor: Siao, Foot Kee, Taman Putri (MY); Loo, Chooi Poon, 41000 Klang, Selangor (MY)
(74) Representative: Winckels, Johannes Hubertus F.

(57) **Abstract**

Air freshener device comprising: a container for housing an air freshener liquid comprising an opening, a neck portion to be inserted in the opening of the container; a wick to be partially submerged in the air freshener liquid and fitted in the neck portion; and a cap section for closing the opening of the container and for uncovering the wick. An upstanding threaded tubular section is provided standing up from the container so that the cap can be unscrewed from the upstanding tubular section, said upstanding threaded tubular section comprising sideways openings. The cap comprises further a central screw cap element encompassing and cooperating with the upstanding threaded tubular section of the central ring, said screw cap element having a cap top section comprising openings for ventilating the wick and outer upstanding walls encompassing the screw cap element and having curved top edges.

## Description

The invention relates to an air freshener device comprising a container for housing an air freshener liquid comprising an opening, a neck portion to be inserted in the opening of the container; a wick to be partially submerged in the air freshener liquid and fitted in the neck portion; and a cap section for closing the opening of the container and for uncovering the wick, the cap section comprising: a central ring to be fitted around the opening of the container and a cap that can be unscrewed relative to the central ring.

Such an arrangement is common for wick type air freshener devices that are to be placed in rooms for providing fragrances.

One type of air freshener is known from the international publication WO2004/110559. Here an air freshener is shown having a relative large central section which is to be submerged in the container for housing a screw element for uncovering the wick. In use the air freshener must be unscrewed to provide fragrances. By controlling the axial movement of the cap sideways openings can be uncovered for adjusting a predetermined dose level.

The invention has as one of its objects to provide a compacter device which does not need a submerged central section for unscrewing the cap. The invention has as one of its further objects to provide a device wherein, in use, the air freshener provides a certain predetermined base level with the cap in closed position.

To this end, the invention offers an air freshener device according to the features of claim 1. In particular, the invention provides an air freshener device, wherein the cap section comprises an upstanding threaded tubular section standing up from the container so that the cap can be unscrewed from the upstanding tubular section, said upstanding threaded tubular section comprising sideways openings; and wherein the cap comprises a central screw cap element encompassing and cooperating with the upstanding threaded tubular section of the central ring, said screw cap element having a cap top section comprising openings for ventilating the wick and outer upstanding walls encompassing the screw cap element and having curved top edges.

By the device according to the invention, the openings are positioned away from the container and uncovered by the cap while fixing the openings relative to the container. In addition, in use, through the openings in the cap top a base ventilation dose is provided.

The invention will be further elucidated referring to the drawings.
Figure 1 shows a schematic side view of the air freshener device according to the invention; and
Figure 2 shows a detailed sectional view of the cap section in open and in closed position.

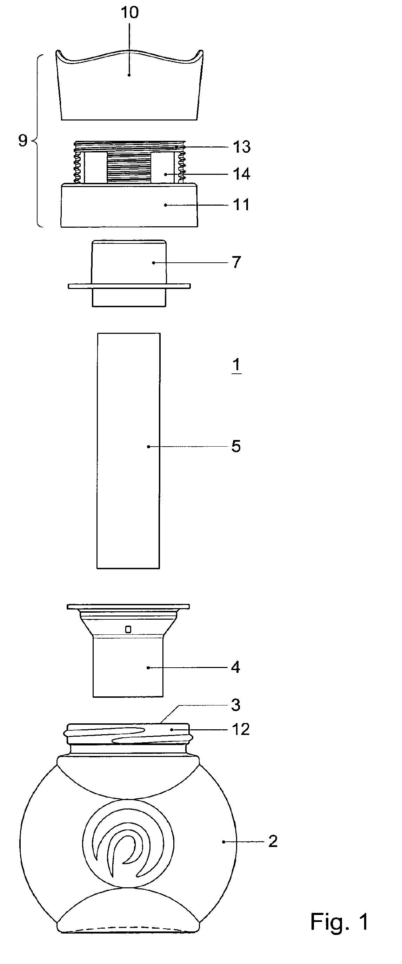

Referring to Figure 1 there is disclosed an air freshener 1 comprising a container 2 for housing an air freshener liquid. The container 2 comprises an opening 3 wherein a neck portion 4 can be inserted. The neck portion 4 is for holding a wick 5 to be partially submerged in the air freshener liquid 6.

The examplary embodiment of Figure 1 further comprises a detachable cap 7 covering the wick 5 and closing the container 2. The cap element can be disposed while the air freshener 8 is in use.

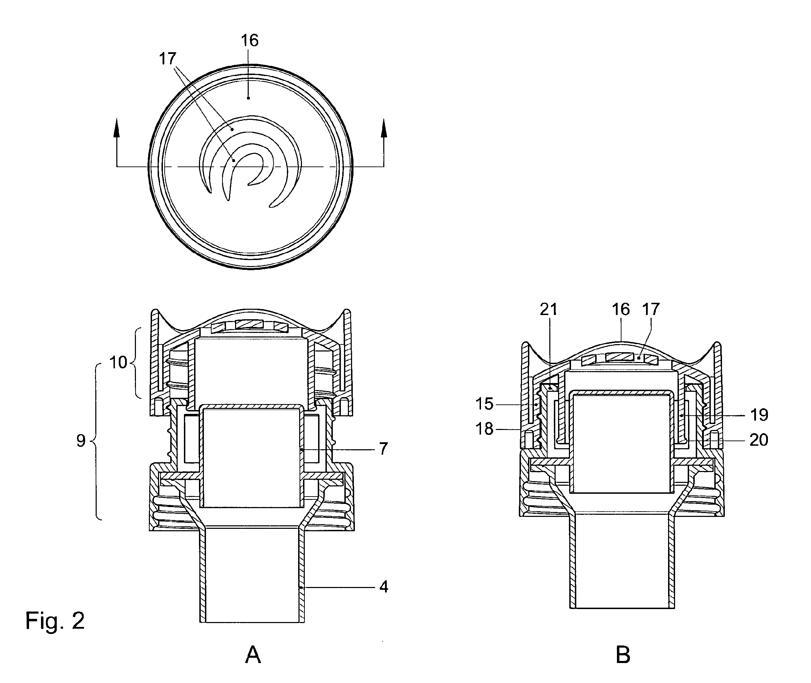

Figure 1 further shows an exploded view of the cap section 9, which, as is apparent from Figure 2, in use, is coupled to a single piece. The cap section 9 closes the opening of the container 2 and uncovers the wick 5 by unscrewing the crown section 10. The cap section 9 comprises a central ring 11 to be screwed on a neck 12 of the container 2. The crown section 10 can be unscrewed relative to the central ring 11.

Figure 1 further shows that the central ring 11 comprises an upstanding threaded tubular section 13 standing up from the container 2 so that the crown section 10 can be unscrewed from the upstanding tubular section 13. The upstanding threaded tubular section 13 comprising sideways openings 14 for ventilating the wick 5 in a dosed manner. By unscrewing the cap the openings can be opened more or less according to a desired dose level.

Referring to Figure 2 the cap section 9 is shown in an opened position (left figure A) and in a closed position (right figure B). The cap can be brought from an opened to a closed position by screwing the crown section 10. The crown section 10 comprises a central screw cap element 15 encompassing and cooperating with the upstanding threaded tubular section 13 of the central ring 11. The screw cap element 15 has a cap top section 16 comprising openings 17 for ventilating the wick 5. These openings are for providing a permanent ventilation to the wick 5 when in use. Furthermore, the crown section 10 has outer upstanding walls 18 encompassing the screw cap element 15 and having curved top edges.

The crown section 10 is fixed relative to the tubular section 13 by an inner cilindrical element 19 that protrudes from the cap top 16. Accordingly the cilindrical element 19 defines a guide for guiding the axial movement of the crown section 10 and encloses the wick 5. The inner cilindrical element 19, on the distal end thereof, comprises outward flanges 20 for stopping the cilinder relative to inward oriented flanges 21 protruding from the upstanding tubular section 13. The central screw cap element, the outer upstanding walls and the cilindrical element are monolithically formed.

In Figure 2 the detachable cap 7 is shown while the device 1 is not in use. The cap 7 is arranged to fit inside the cilindrical element 19 and seal the container and the wick to be able to store and transport the air freshener device 1.

While the invention has been described with reference to the drawings, the invention is not limited thereto but may encompass modifications and variations that fall within the scope of the invention as defined in the annexed claims.

## Claims

1. Air freshener device comprising:
- a container for housing an air freshener liquid comprising an opening,
- a neck portion to be inserted in the opening of the container;
- a wick to be partially submerged in the air freshener liquid and fitted in the neck portion; and
- a cap section for closing the opening of the container and for uncovering the wick, the cap section comprising:
- a central ring to be fitted around the opening of the container and
- a cap that can be unscrewed relative to the central ring, wherein the central ring comprises:
- an upstanding threaded tubular section standing up from the container so that the cap can be unscrewed from the upstanding tubular section, said upstanding threaded tubular section comprising sideways openings; and wherein the cap comprises:
- a central screw cap element encompassing and cooperating with the upstanding threaded tubular section of the central ring, said screw cap element having a cap top section comprising openings for ventilating the wick and
- outer upstanding walls encompassing the screw cap element and having curved top edges.

2. Air freshener according to claim 1, wherein the screw cap element comprises: an inner cilindrical element protruding from the cap top, the cilindrical element defining a guide for guiding the axial movement of the screw cap and for enclosing the wick, said inner cilindrical element comprising outward flanges for stopping the cilinder relative to inward oriented flanges protruding from the upstanding tubular section;

3. Air freshener according to claim 2, further comprising a detachable cap element covering the wick and closing the container, the cap element arranged to fit inside the cilindrical element.

4. Air freshener according to claim 2 or 3, wherein the central screw cap element, the outer upstanding walls and the cilindrical element are monolithically formed.
